# EUROPEAN PATENT SPECIFICATION

(11) **EP 4 674 339 B1**
(45) Date of publication and mention of the grant of the patent: **14.10.2026**
(21) Application number: 25218126.8
(22) Date of filing: 30.03.2023
(51) Int. Cl.: A61B 3/032, A61B 3/10

(54) **VISUAL ACUITY MEASUREMENT IN VR HEADSET**
SEHSCHÄRFEMESSUNG IN VR-KOPFHÖRER
MESURE DE L'ACUITÉ VISUELLE DANS UN CASQUE DE RÉALITÉ VIRTUELLE

(30) Priority: 31.03.2022 US 202263325963 P
(43) Date of publication of application: 07.01.2026
(62) Document number(s) of the earlier application(s) in accordance with Art. 76 EPC: 23718925.3 / 4 498 892
(73) Proprietor: Verily Health Inc., Dallas, Texas 75019 (US)
(72) Inventor: SINHA, Supriyo, San Francisco, 94080 (US); LEINDECKER, Nick, San Francisco, 94080 (US); CHAN, Jeremy, San Francisco, 94080 (US); AZAR, Dimitri, San Francisco, 94080 (US)
(74) Representative: Mewburn Ellis LLP

(56) References cited:
- US-A1- 2021 089 118
- US-A1- 2021 330 185

## Description

### Related Applications

This patent application claims the benefit of the earlier filing date of US provisional no. 63/325,963 filed 31 Mar 2022.

### Field

The subject matter of this disclosure relates to techniques for measuring visual acuity of a human user, using a virtual reality, VR, headset worn by the user.

### Background

Visual acuity is one of the most common measurements done at the office of an eye care professional (ECP). Eye examinations have been suggested to be performed with a head-worn VR unit. The user puts on the VR head unit, and the ECP operates software that configures the display inside the VR head unit to display various visual elements whose shape, color or size is selected for the test. The user provides feedback to the ECP on how they perceive the visual elements.

US 2021/089118 A1 discloses a VR headset system for measuring visual acuity, refraction, and aberrations. The system includes two optical heads (one per eye) with a detailed screen for high-resolution visual acuity testing and a background screen for peripheral vision. The system incorporates pupil cameras for eye tracking, NIR illumination, and beam splitters for directing light paths between displays, eye, and cameras.

US 2021/330185 A1 discloses a vision assessment and therapy device including a VR-HMD unit with eye tracking, accommodation measurement, and varifocal optics. It focuses on therapy applications.

### Summary

The present invention is defined in claim 1 while preferred embodiments are set forth in the dependent claims.

An aspect of the disclosure here is a method for measuring visual acuity to at least the 20/20 level with an otherwise relatively standard consumer-grade VR headset that has been modified as described below. A standard consumer-grade VR headset does not have sufficient full frame pixel resolution in its display to measure acuity close to the 20/20 level. In accordance with one aspect of the disclosure here, central visual acuity of the wearer can be performed using the following system. A headset includes a first display, a second display that is smaller than the first display and has lower pixel count than the first display, and a dichroic filter positioned to pass visible light from the first display toward the eye and reflect visible light from the second display toward the eye. Pixel data control circuitry is coupled to darken the first display while simultaneously activating the second display to display a pattern for testing visual acuity, responsive to a first visual acuity test selection.

The above summary does not include an exhaustive list of all aspects of the present disclosure. It is contemplated that the disclosure includes all systems and methods that can be practiced from all suitable combinations of the various aspects summarized above, as well as those disclosed in the Detailed Description below and particularly pointed out in the Claims section. Such combinations may have particular advantages not specifically recited in the above summary.

### Brief Description of the Drawings

Several aspects of the disclosure here are illustrated by way of example and not by way of limitation in the figures of the accompanying drawings, in which like references indicate similar elements. It should be noted that references to "an" or "one" aspect in this disclosure are not necessarily to the same aspect, and they mean at least one. Also, in the interest of conciseness and reducing the total number of figures, a given figure may be used to illustrate the features of more than one aspect of the disclosure, and not all elements in the figure may be required for a given aspect.
Fig. 1 depicts a user wearing a VR headset.
Fig. 2 is a block diagram of an example system for measuring visual acuity using a VR headset that has an eyetracker camera.
Fig. 3 illustrates an example transmission spectrum for the first dichroic filter used in the system of Fig. 2.
Fig. 4 shows an example transmission spectrum for a first version of the second dichroic filter used in a first version of the system of Fig. 2.
Fig. 5 shows an example transmission spectrum for a second version of the second dichroic filter used in a second version of the system of Fig. 2.
Fig. 6 shows an example transmission spectrum for another version of the first dichroic filter.
Fig. 7 is a block diagram of an example system for measuring visual acuity using a VR headset that does not have an eyetracker camera.

### Detailed Description

Several aspects of the disclosure with reference to the appended drawings are now explained. Whenever the shapes, relative positions and other aspects of the parts described are not explicitly defined, the scope of the invention is not limited only to the parts shown, which are meant merely for the purpose of illustration. Also, while numerous details are set forth, it is understood that some aspects of the disclosure may be practiced without these details. In other instances, well-known circuits, structures, and techniques have not been shown in detail so as not to obscure the understanding of this description.

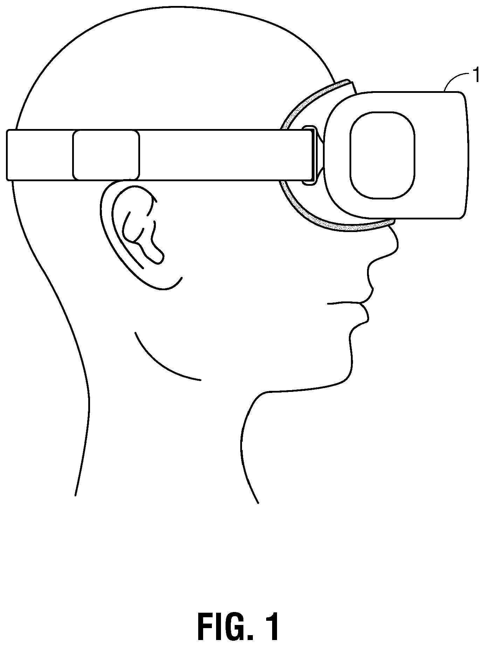

Fig. 1 depicts a user (wearer) wearing a virtual reality, VR, headset which as modified below can be used to perform various ophthalmic examinations on the eyes of the wearer, including visual acuity testing for 20/20 vision or better. The modifications enable the use of a relatively low-cost solution in the form of an otherwise consumer grade VR headset.

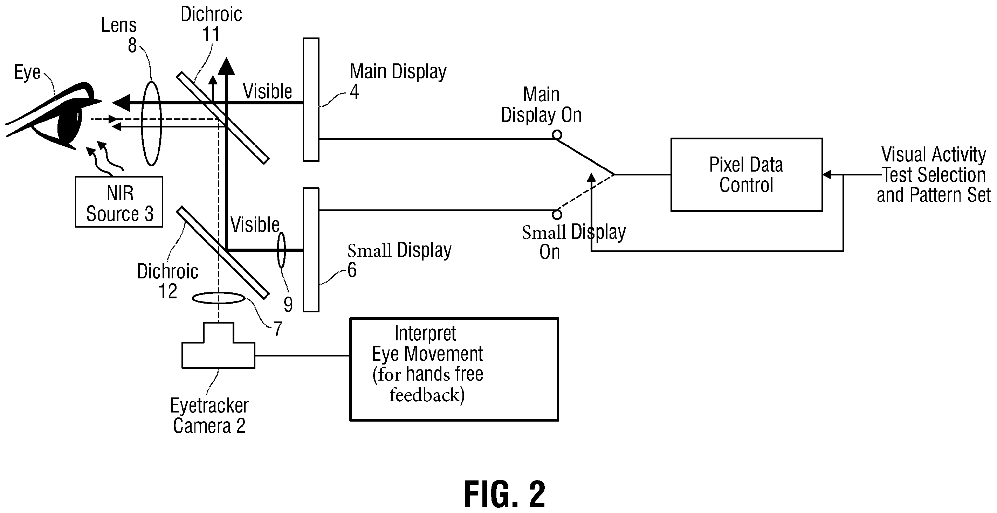

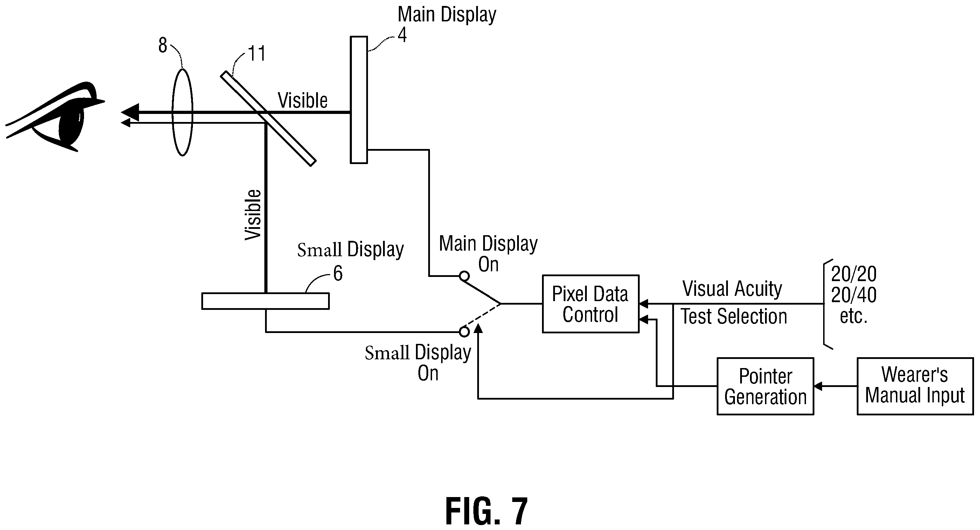

Fig. 2 is a block diagram of an example system for measuring visual acuity using a VR headset, and in particular one that has an eyetracker camera 2 aimed at an eye of the wearer (also referred to here as an eye tracking headset). The eyetracker camera 2 is an imaging device whose image data can be processed by eyetracking software (that may be executed by a processor in the VR headset) to measure movement of the wearers eye and track the direction of their gaze in real-time. With the addition of electronics referred to here as eye movement interpretation logic that processes the image data produced by the eyetracker camera, the system enables hands-free feedback from the wearer of the headset during visual acuity testing. This is in contrast to the example VR headset shown in Fig. 7 and described further below which can also perform visual acuity testing but with manual or audible wearer feedback, as it does not have an eyetracker camera.

Integrated within a headset housing of the system in Fig. 2 is an illumination source 3, such as one or more near infrared, NIR, light emitting diodes that produces NIR light, which illuminates an eye of the wearer of the headset as shown. This NIR light is reflected off the eye and then travels through a lens 8, followed by being reflected off a first dichroic filter 11 in the direction of an imaging lens 7 that forms an NIR image for pick up by the eyetracker camera 2. In doing so, the reflected NIR light passes through a second dichroic filter 12, to be described below.

The first dichroic filter 11 is angled relative to a path taken by visible light that is emitted from a first display 4 and that passes through the first dichroic filter 11, before impinging on the eye. The first display 4 may be a main display of the headset, which serves to present virtual reality images to the eye. The first dichroic filter 11 is also angled so that it can reflect the NIR light that has been reflected from the eye, towards the second dichroic filter 12. The second dichroic filter 12 is positioned to pass the reflected NIR light from the first dichroic filter 11 towards the eyetracker camera 2, but it is also angled relative to that path so as to reflect visible light emitted from a second display 6 towards the first dichroic filter 11. The second display 6 may be a microdisplay. More generally, a display surface of the first display 4 may be at least two times larger than that of the second display 6, and the first display 4 has at least twice the full frame pixel count of the second display 6. The first display 4 may for example have a full frame pixel count of 1920x1080 pixels or more, while the second display 4 may for example have a full frame pixel count of less than 2000 pixels, e.g., 60x32 dots, or even less than 1000 pixels. As explained next, the smaller and lower pixel count of the second display 6 enables a low cost solution for visual acuity testing at 20/20 vision or better. Central visual acuity testing using a variation of the typical Snellen test or Random E test (that is adapted to the distance between the eye and the second display 6 within a VR headset) can be performed using the smaller and lower pixel count second display 6. This capability would be in addition to a number of other ophthalmic examinations that would be performed using the VR headset, and in particular using the larger, first display 4 to display patterns for those other examinations.

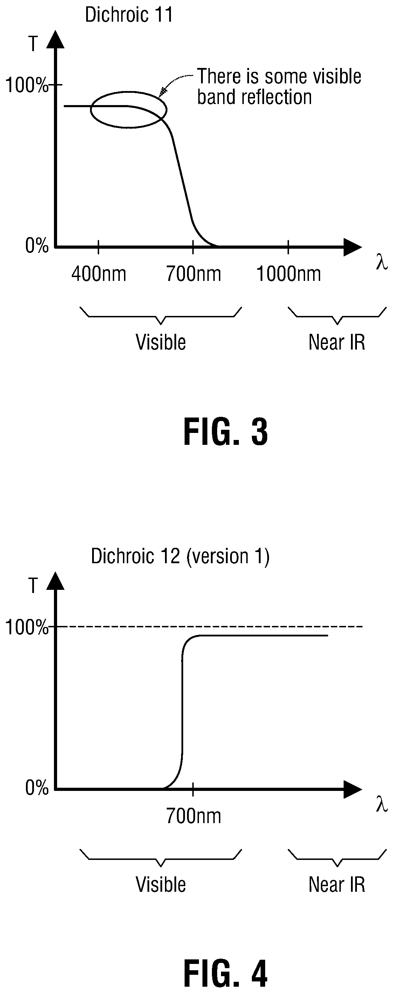

In one aspect of the disclosure here, the system in Fig. 2 operates based on the fact that the first dichroic filter 11 while having primary or strong transmission in the visible spectrum also exhibits a weak or small amount of reflection (in the visible spectrum.) This small reflection in the visible spectrum may be enough to direct a testing pattern that is emitted from the second display 6 to be seen by the eye of the wearer. Fig. 3 shows an example transmission spectrum for the first dichroic filter 11. This transmission is not 100% or perfect as indicated, because there is some relatively small amount of visible band reflection. For example, the visible band reflection may be more than zero but less than ten percent reflection, across a full range of visible colors, e.g., in the visible spectrum spanning from 380 nm to 700 nm. In another example of the transmission spectrum in Fig. 3, the first dichroic filter 11 would exhibit between five percent to ten percent reflection of the visible light. As to the NIR band, Fig. 3 also shows how the first dichroic filter 11 has almost no transmission in the NIR region, as NIR is being strongly reflected.

Fig. 4 shows an example transmission spectrum for a first version of the second dichroic filter 12, that works in a first version of the system exactly as shown in Fig. 2. In this first version, the second dichroic filter 12 exhibits almost no transmission (or almost perfect reflection) of the full range of visible colors up to about 700 nm, but then almost complete transmission in the NIR region.

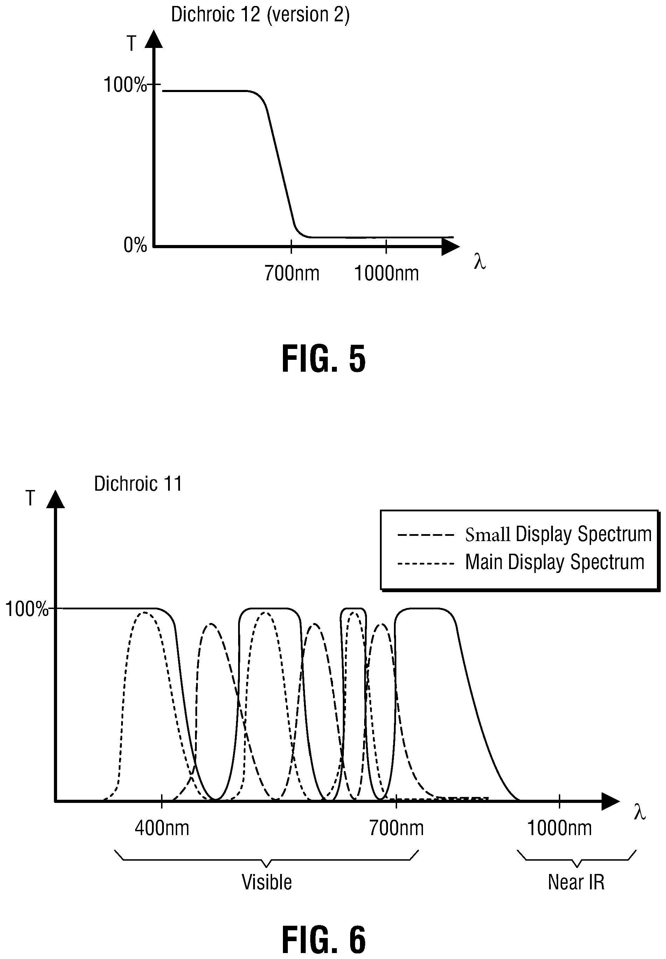

Fig. 5 shows an example transmission spectrum for another version of the second dichroic filter 12 that is suitable for use in another version of the system of Fig. 2. In this other version of the system, the locations of the second display 6 and the eyetracker camera 2 are swapped relative to how they are shown in Fig. 2. As a result, the transmission spectrum of the second version of the second dichroic filter 12 is flipped about approximately 700 nm, relative to the first version shown in Fig. 4: the second version reflects NIR light (from the first dichroic filter 11 towards the eyetracker camera 2), and passes the full range of visible light (from the second display 6 to the first dichroic filter 11.)

Still referring to the system in Fig. 2, given that the visible band reflection characteristic of the first version of the first dichroic filter 11 shown in Fig. 3 is weak (relative to its visible band transmission characteristic), the testing pattern produced by the second display 6 might be difficult to see for the wearer. As a result, there may be a need to darken the first display 4 while the second display 6 is active, to improve the ability of the wearer to see the testing pattern that is being produced by the second display 6. For this reason, pixel data control circuitry may be added as shown in Fig. 2, that is coupled to darken the first display 4 while simultaneously activating the second display 6 to display the pattern for visual acuity testing. This would be in response to a first visual acuity test selection whose image pattern may be designed for testing 20/20 vision or better which may require use of the second display 6. However, when testing for 20/40 vision, 20/60 vision, or worse, the first display 4 may be better than the second display 6 at showing larger patterns: in that case, the pixel data control circuitry may darken the second display 6 while simultaneously activating the first display 4 to display a pattern for testing 20/40 vision or worse. The term "darken" as used here means to power off the display, activate the display with only black pixels in the full frame, or activate the display to show a pixel pattern that is dark enough to not interfere with testing the wearer's visual acuity using the other display (which is showing the visual acuity test pattern.) More generally, there may be a selection between the first display 4 (e.g., a main, larger, high pixel count display) and the second display 6 (e.g., a "microdisplay", or smaller, low pixel count display), according to any one of two or more visual acuity test selections, and the pixel data control circuitry provides the selected display with the appropriate pixel pattern set for each test selection.

As mentioned above, in the system of Fig. 2 the first dichroic filter 11 serves to reflect toward the eye the visual acuity pattern that is being displayed by the second display 6. This visible band reflection may be across the full visible spectrum but it is a weak or small reflection, as seen in the transmission curve shown in Fig. 3. As such, the testing pattern produced by the second display 6 might be difficult to see by the wearer due to a ghosting effect that arises from the weak reflection characteristic. A dichroic filter has two surfaces, and so the reflection from each of these surfaces may be specified or considered in the design of the system. If the dichroic filter is thin, e.g., effectively a 'pellicle', or the second surface is assumed to be perfectly anti-reflection, AR, coated, then advantageously an undesired and offset 'ghost' reflection may not be evident to the wearer of the headset.

To address the ghosting problem, Fig. 6 shows an example transmission spectrum for another version of the first dichroic filter 11 (that is suitable for use in both versions of the system of Fig. 2.) This second version of the dichroic filter 11 enables the image produced by the second display 6 to be reflected with greater fidelity (than would be achieved using the transmission spectrum shown in Fig. 3.) Here, the transmission characteristic in the visible spectrum is not "uniform" across the full visible range, but instead varies in a way that exhibits notches. In other words, the transmission characteristic has a bandpass characteristic; it contains several pass bands which are aligned with the discrete colors, respectively, that are emitted by the first display 4. The transmission notches are regions in which the dichroic filter 11 reflects strongly (e.g., at least 50%.)

In the example shown in Fig. 6, the first dichroic filter 11 has three pass bands that are aligned with a first visible color being a shade of blue, a second visible color being a shade of green, and a third visible color being a shade of red, respectively. Those are a common set of primary, visible band colors that are emitted by the first display 4 when producing a full color or black and white image. The first display 4 may be implemented as an organic light emitting diode display that has a color filter array or color filter method having those three primary colors. Tuning the transmission characteristic of the first dichroic filter 11 in this manner results in strong transmission (by the first dichroic filter 11) of the image produced by the first display 4.

Now, in order to also achieve strong reflection (by the first dichroic filter 11) of the visible image produced by the second display 6, the second display 6 may also be implemented as one that emits in generally the same primary or discrete colors as the first display 4, but those primary colors are slightly shifted (in wavelength) relative to the primary colors emitted by the first display 4. More generally, the color filter array (method) of the second display 4 may have a fourth visible color, a fifth visible color and a sixth visible color, which in the example of Fig. 6 are different shades of blue, green and red, respectively. As seen in Fig. 6, these colors are aligned with the notch bands of the transmission characteristic of the first dichroic filter 11, which results in strong reflection (by the first dichroic filter 11) of the visible band image produced by the second display 6. Of course, the choice of RGB colors shown in Fig. 6 is only an example - the concept is also applicable to displays that might emit in a different combination of primary colors. As a dichroic filter can also have a polarization dependent transmission and reflection profile, the headset can be designed to optimally account for this. The first display's polarization state will likely not be modified as it is the primary display for the headset, however a polarizer and/or waveplate could be placed in the path of the image output from the second display to optimize the reflection of the second display's output from the first dichroic filter 11.

The system in Fig. 2 described above has an eyetracker camera 2 that enables hands-free feedback from the wearer, during ophthalmic examination of the eyes of the wearer. Fig. 7 is a block diagram of a system for measuring visual acuity in which the VR headset does not have the eyetracker camera 2. As such, feedback from the wearer about what they see during ophthalmic examination (while wearing the VR headset) is provided by the wearer either via manual input such as through a joystick or physical button press, or via audible input. The headset has the first display 4, the second display 6 that is smaller than the first display and has lower pixel count than the first display 4, and the first dichroic filter 11. The first dichroic filter 11 is configured and positioned to (strongly, e.g., with at least 70% transmission) pass visible light from the first display 4 toward the eye and weakly reflect, e.g., with no more than 20% reflection, or with no more than 5% reflection, the visible light from the second display 6 toward the eye. There may also be pixel data control circuitry coupled to darken the first display 4 while simultaneously activating the second display 6 to display a pattern for testing visual acuity, especially when testing for 20/20 vision, responsive to a first visual acuity test selection. Many of the other features and processes described above in connection with Fig. 3 and Fig. 6 apply to the version of the VR headset shown in Fig. 7.

## Claims

1. A system for measuring visual acuity, the system comprising:
a headset (1) that comprises:
a first display (4);
a second display (6) that is smaller than the first display (4) and has a lower full frame pixel resolution than the first display (4); and
a dichroic filter (11) positioned to pass visible light from the first display (4) toward an eye and reflect visible light from the second display (6) toward the eye, wherein the dichroic filter (11) is configured to pass visible light from the first display (4) with at least 70% transmission and reflect light from the second display (6) with no more than 20% reflection; and
pixel data control circuitry configured to darken the first display (4) while simultaneously activating the second display (6) to display a pattern for testing visual acuity, responsive to a first visual acuity test selection.

2. The system of claim 1 wherein a display surface of the first display (4) is at least two times larger than that of the second display (6), and the first display (4) has at least twice a full frame pixel resolution of the second display (6).

3. The system of claim 1 wherein the dichroic filter (11) has more than zero but less than ten percent reflection of visible light across a visible spectrum spanning 380 to 700 nanometers.

4. The system of claim 3 wherein the dichroic filter (11) has more than five percent reflection of visible light.

5. The system of claim 1 wherein:
the first display (4) comprises a first color filter array having a first visible color, a second visible color and a third visible color, the dichroic filter (11) having a plurality of pass bands that are aligned with the first visible color, the second visible color and the third visible color, respectively, and
the second display (6) comprises a second color filter array having a fourth visible color, a fifth visible color and a sixth visible color, the dichroic filter (11) having a plurality of notch bands that are aligned with the fourth visible color, the fifth visible color and the sixth visible color, respectively.

6. The system of claim 5, wherein the first visible color and the fourth visible color are shades of blue, the second visible color and the fifth visible color are shades of green, and the third visible color and the sixth visible color are shades of red.

7. The system of claim 1, wherein the pixel data control circuitry is coupled to the first (4) and second (6) displays.

## Patentansprüche

1. System zum Messen von Sehschärfe, wobei das System Folgendes umfasst:
ein Headset (1), das Folgendes umfasst:
eine erste Anzeige (4);
eine zweite Anzeige (6), die kleiner ist als die erste Anzeige (4) und eine niedrigere Vollformat-Pixelauflösung als die erste Anzeige (4) aufweist; und
einen dichroitischen Filter (11), der angeordnet ist, um sichtbares Licht von der ersten Anzeige (4) in Richtung eines Auges durchzulassen und um sichtbares Licht von der zweiten Anzeige (6) in Richtung des Auges zu reflektieren, wobei der dichroitische Filter (11) ausgelegt ist, um sichtbares Licht von der ersten Anzeige (4) mit zumindest 70 % Transmission durchzulassen und Licht von der zweiten Anzeige (6) mit nicht mehr als 20 % Reflexion zu reflektieren; und
eine Pixeldatensteuerschaltungsanordnung, die ausgelegt ist, um die erste Anzeige (4) zu verdunkeln, und um gleichzeitig die zweite Anzeige (6) zu aktivieren, um ein Muster zum Prüfen der Sehschärfe als Reaktion auf die Auswahl eines ersten Sehschärfetests anzuzeigen.

2. System nach Anspruch 1, wobei eine Anzeigeoberfläche der ersten Anzeige (4) zumindest zweimal größer ist als jene der zweiten Anzeige (6), und wobei die erste Anzeige (4) eine Vollformat-Pixelauflösung aufweist, die zumindest zweimal höher ist als jene der zweiten Anzeige (6).

3. System nach Anspruch 1, wobei der dichroitische Filter (11) mehr als null, aber weniger als 10 % Reflexion von sichtbarem Licht über ein sichtbares Spektrum, das 380 bis 700 nm überspannt, aufweist.

4. System nach Anspruch 3, wobei der dichroitische Filter (11) mehr als 5 % Reflexion von sichtbarem Licht aufweist.

5. System nach Anspruch 1, wobei:
die erste Anzeige (4) eine erste Farbfilteranordnung umfasst, die eine erste sichtbare Farbe, eine zweite sichtbare Farbe und eine dritte sichtbare Farbe aufweist, wobei der dichroitische Filter (11) eine Vielzahl von Durchlassbereichen aufweist, die an der ersten sichtbaren Farbe, der zweiten sichtbaren Farbe bzw. der dritten sichtbaren Farbe ausgerichtet sind, und
die zweite Anzeige (6) eine zweite Farbfilteranordnung umfasst, die eine vierte sichtbare Farbe, eine fünfte sichtbare Farbe und eine sechste sichtbare Farbe aufweist, wobei der dichroitische Filter (11) eine Vielzahl von Bandsperrbereichen aufweist, die an der vierten sichtbaren Farbe, der fünften sichtbaren Farbe bzw. der sechsten sichtbaren Farbe ausgerichtet ist.

6. System nach Anspruch 5, wobei die erste sichtbare Farbe und die vierte sichtbare Farbe Blauschattierungen sind, die zweite sichtbare Farbe und die fünfte sichtbare Farbe Grünschattierungen sind und die dritte sichtbare Farbe und die sechste sichtbare Farbe Rotschattierungen sind.

7. System nach Anspruch 1, wobei die Pixeldatensteuerschaltungsanordnung mit der ersten (4) und der zweiten (6) Anzeige gekoppelt ist.

## Revendications

1. Système de mesure de l'acuité visuelle, le système comprenant :
un casque (1) qui comprend :
un premier dispositif d'affichage (4) ;
un second dispositif d'affichage (6) qui est plus petit que le premier dispositif d'affichage (4) et qui présente une résolution de pixels de trame complète inférieure à celle du premier dispositif d'affichage (4) ; et
un filtre dichroïque (11) positionné pour laisser passer de la lumière visible depuis le premier dispositif d'affichage (4) vers un œil et réfléchir de la lumière visible depuis le second dispositif d'affichage (6) vers l'œil, dans lequel le filtre dichroïque (11) est configuré pour laisser passer de la lumière visible depuis le premier dispositif d'affichage (4) avec une transmission d'au moins 70 % et réfléchir de la lumière depuis le second dispositif d'affichage (6) avec une réflexion ne dépassant pas 20 % ; et
des circuits de commande de données de pixel configurés pour assombrir le premier dispositif d'affichage (4) tout en activant simultanément le second dispositif d'affichage (6) pour afficher un motif pour tester l'acuité visuelle, en réponse à une première sélection de test d'acuité visuelle.

2. Système selon la revendication 1, dans lequel une surface d'affichage du premier dispositif d'affichage (4) est au moins deux fois plus grande que celle du second dispositif d'affichage (6), et le premier dispositif d'affichage (4) présente au moins deux fois une résolution de pixels de trame complète du second dispositif d'affichage (6).

3. Système selon la revendication 1, dans lequel le filtre dichroïque (11) présente une réflexion de lumière visible supérieure à zéro mais inférieure à dix pour cent sur un spectre visible s'étendant de 380 à 700 nanomètres.

4. Système selon la revendication 3, dans lequel le filtre dichroïque (11) présente une réflexion de lumière visible supérieure à cinq pour cent.

5. Système selon la revendication 1, dans lequel :
le premier dispositif d'affichage (4) comprend un premier réseau de filtres de couleur présentant une première couleur visible, une deuxième couleur visible et une troisième couleur visible, le filtre dichroïque (11) présentant une pluralité de bandes passantes qui sont alignées avec la première couleur visible, la deuxième couleur visible et la troisième couleur visible, respectivement, et
le second dispositif d'affichage (6) comprend un second réseau de filtres de couleur présentant une quatrième couleur visible, une cinquième couleur visible et une sixième couleur visible, le filtre dichroïque (11) présentant une pluralité de bandes à encoche qui sont alignées respectivement avec la quatrième couleur visible, la cinquième couleur visible et la sixième couleur visible.

6. Système selon la revendication 5, dans lequel la première couleur visible et la quatrième couleur visible sont des nuances de bleu, la deuxième couleur visible et la cinquième couleur visible sont des nuances de vert, et la troisième couleur visible et la sixième couleur visible sont des nuances de rouge.

7. Système selon la revendication 1, dans lequel les circuits de commande de données de pixel sont couplés aux premier (4) et second (6) dispositifs d'affichage.
